# EUROPEAN PATENT APPLICATION

(11) **EP 3 199 153 A1**
(43) Date of publication of application: **02.08.2017**
(21) Application number: 15844075.0
(22) Date of filing: 28.09.2015
(51) Int. Cl.: A61K 31/423, A61K 31/455, A61P 3/06, A61P 9/10, A61P 43/00

(54) **DYSLIPIDEMIA THERAPEUTIC AGENT**

(30) Priority: 26.09.2014 JP 2014196002
(71) Applicant: Kowa Company, Ltd., Nagoya-shi, Aichi-ken 460-8625 (JP)
(72) Inventor: INOKUCHI, Yuta, Higashimurayama-shi Tokyo 189-0022 (JP); TAKIZAWA, Toshiaki, Higashimurayama-shi Tokyo 189-0022 (JP)
(74) Representative: Adam, Holger
(86) International application number: PCT/JP2015/077235
(87) International publication number: WO 2016/047800

(57) **Abstract**

This invention provides a combination-drug composition and a combination use of pharmaceuticals for preventing and/or treating dyslipidemic conditions such as hyper-LDL cholesterolemia in mammals, including humans.

This invention pertains to a drug composition for preventing and/or treating dyslipidemia and the like, the drug composition including the following:
(R)-2-[3-[[N-(benzoxazol-2-yl)-N-3-(4-methoxyphenoxy)propyl ]aminomethyl]phenoxy] butyric acid, a salt thereof, or a solvate of either; and nicotinic acid and nicotinic-acid amide, collectively referred to as niacin, an ester derivative thereof, a salt thereof, or a solvate of any of these.

## Description

### Technical Field

The present invention relates to a composition containing (R)-2-[3-[[N-(benzoxazol-2-yl)-N-3-(4-methoxyphenoxy)propyl ]aminomethyl]phenoxy] butyric acid, and nicotinic acid, nicotinic acid amide, which are collectively referred to as niacin, an ester derivative thereof, or the like, and combination use thereof for preventing and/or treating dyslipidemic conditions such as atherosclerosis or hypercholesterolemia.

### Background Art

In recent years, due to westernization of food, patients with hypercholesterolemia, hypertriglyceridemia, hypo HDL cholesterolemia, or the like, which are in a category of so-called lifestyle-related diseases have been increasing. In addition, recently, patients with mixed or combined dyslipidemia with both hypercholesterolemia and hypertriglyceridemia have been increasing. Particularly, patients of mixed dyslipidemia have LDL cholesterol (LDL-C) and triglyceride (TG) raised, and have HDL cholesterol (HDL-C) lowered. Such a high TG and low HDL-C condition is observed also in patients with metabolic syndrome or diabetes. It has been proven that hyper LDL cholesterolemia, hypo HDL cholesterolemia, and hypertriglyceridemia are risk factors of a coronary artery disease (CAD), a cerebrovascular disorder, or the like. The importance of the management of dyslipidemia is described in "Guidelines for Prevention of Atherosclerotic Cardiovascular Diseases 2012" released by Japan Atherosclerosis Society.

Dyslipidemia, particularly hypercholesterolemia has become a disease having a quite high degree of medical satisfaction due to advent of statins. However, from results of many large-scale clinical trials, it has been found that further lowering of LDL cholesterol in blood leads to prevention of coronary artery diseases (the lower, the better). More strict lipid control has been recommended. Many patients cannot reach the intended LDL-C level in blood only with statins. Combination use of multiple pharmaceutical agents has been also required (Non-Patent Document 1).

PPAR is one of receptors belonging to a nuclear receptor family. Existence of three subtypes (α, γ, and δ) is known for this receptor (Non-Patent Document 2). Among these types, PPARα is mainly expressed in the liver. When PPARα is activated, production of apo C-III is suppressed, followed by activation of lipoprotein lipase (LPL). As a result, fat is decomposed. As PPARα agonist, for example, unsaturated fatty acids and fibrate pharmaceutical agents such as fenofibrate, bezafibrate, or gemfibrozil have been known (Non-Patent Document 3). In recent years, a compound having a stronger and more selective PPARα activating effect than a conventional fibrate pharmaceutical agent has been reported (Patent Document 1).

Niacin is a water-soluble vitamin belonging to a vitamin B complex and is constituted by nicotinic acid and nicotinic acid amide, and is biosynthesized also in a human body. Niacin is widely present in plants and animals, and is usually ingested through food. Each of Niacin and niacin ester derivatives (niceritrol) acts as a coenzyme (nicotinamide adenine dinucleotide (NAD)) of an oxidation-reduction enzyme in energy metabolism, and has been used for treating carbohydrate-lipid metabolism for many decades (Non-Patent Documents 4 and 5).

Under such circumstances, studies to improve dyslipidemia by combination use of PPARα agonist and niacin have been made. In a study using cholesterol diet load or Triton load hyperlipemia rats, it has been reported that continuous administration of bezafibrate and niacin into duodenal brings about further lowering of total cholesterol and triglyceride and further rising of HDL cholesterol in blood than intermittent oral administration (Non-Patent Document 6). In a study using positive lipemia rats or fructose load hyperlipidemia rats, it has been reported that combination use of clofibrate and nicotinic acid brings about synergistic lowering of triglyceride in blood as compared with administration of each thereof (Non-Patent Document 7). However, in these two reports, the former reports that sustained release of a drug is helpful in improving efficacy, and the latter mainly reports change in pharmacokinetics by combination use. Neither of the Documents describes an effect of lowering LDL cholesterol. On the other hand, it has been reported that combination use of clofibrate and nicotinic acid in sufferers of myocardial infarction lowers total cholesterol and triglyceride in blood to significantly reduce deaths from an ischemic heart disease as compared with a group of only diet for decreasing plasma lipid (Non-Patent Document 8) . However, there is no comparison with an effect by single use of fibrate or nicotinic acid. In addition, an effect of lowering LDL cholesterol has not been confirmed.

### Citation List

### Patent Document

Patent Document 1: WO 2005/023777 A1

### Non-Patent Document

Non-Patent Document 1: Journal of Pharmacological Sciences 129, 267-270(2007)
Non-Patent Document 2: J. Lipid Research, 37, 907-925 (1996)
Non-Patent Document 3: Trends in Endocrinology and Metabolism, 15(7), 324-330(2004)
Non-Patent Document 4: British Journal of Pharmacology, 153, S68-S75(2008)
Non-Patent Document 5: Metabolism, 46(4), 355-358(1997)
Non-Patent Document 6: J Pharm Sci, 89(8), 1046-53(2000)
Non-Patent Document 7: Biochemical Pharmacology, 28, 1163-1167(1979)
Non-Patent Document 8: Atherosclerosis, 37, 129-138(1980)

### Summary of the Invention

### Problems to be Solved by the Invention

An object of the present invention is to provide a pharmaceutical composition comprising a combination of pharmaceutical agents and combination use of pharmaceutical agents for preventing and/or treating dyslipidemic conditions such as atherosclerosis, hypercholesterolemia, or hyper LDL cholesterolemia.

### Means to Solving the Problems

The present inventors made intensive studies in view of these circumstances. As a result, the present inventors have found that a strong effect of lowering LDL cholesterol in blood is exhibited by combination use of niacin and (R)-2-[3-[[N-(benzoxazol-2-yl)-N-3-(4-methoxyphenoxy)propyl ]aminomethyl]phenoxy] butyric acid (Example 85 in Patent Document 1: hereinafter, also referred to as compound A) reported as a selective PPARα activation agent, and thus have completed the present invention.

That is, the present invention provides a pharmaceutical composition for preventing and/or treating dyslipidemia, comprising a) (R)-2-[3-[[N-(benzoxazol-2-yl)-N-3-(4-methoxyphenoxy)propyl ]aminomethyl]phenoxy] butyric acid, a salt thereof, or a solvate thereof, and b) niacin, an ester derivative thereof, a salt thereof, or a solvate thereof.

More specifically, the present invention provides a pharmaceutical composition for preventing and/or treating hyper LDL cholesterolemia, containing a) (R)-2-[3-[[N-(benzoxazol-2-yl)-N-3-(4-methoxyphenoxy)propyl ]aminomethyl]phenoxy] butyric acid, a salt thereof, or a solvate thereof, and b) niacin, an ester derivative thereof, a salt thereof, or a solvate thereof.

More specific description of the present invention is as follows.
(1) A pharmaceutical composition for preventing and/or treating dyslipidemia, comprising (R)-2-[3-[[N-(benzoxazol-2-yl)-N-3-(4-methoxyphenoxy)propyl ]aminomethyl]phenoxy] butyric acid (compound A), a salt thereof, or a solvate of either, and niacin, an ester derivative thereof, a salt of either, or a solvate of any of these.
(2) The pharmaceutical composition described in (1), in which the dyslipidemia is hyper LDL cholesterolemia (LDL-C).
(3) The pharmaceutical composition described in (1) or (2), in which a mass ratio between compound A, a salt thereof, or a solvate of either, and niacin, an ester derivative thereof, a salt of either, or a solvate of any of these is from 1:1 to 1:10000.
(4) The pharmaceutical composition described in any one of (1) to (3), in which niacin is nicotinic acid.
(5) The pharmaceutical composition described in any one of (1) to (3), in which niacin is nicotinic acid amide.
(6) A pharmaceutical composition for lowering LDL cholesterol (LDL-C), comprising compound A, a salt thereof, or a solvate of either, and niacin, an ester derivative thereof, a salt of either, or a solvate of any of these.
(7) The pharmaceutical composition described in (6), in which a disease requiring lowering of LDL cholesterol (LDL-C) is hyper LDL cholesterolemia (LDL-C).
(8) The pharmaceutical composition described in (6) or (7), in which niacin is nicotinic acid.
(9) The pharmaceutical composition described in (6) or (7), in which niacin is nicotinic acid amide.
(10) The pharmaceutical composition described in any one of (6) to (9), in which a mass ratio between compound A, a salt thereof, or a solvate of either; and niacin, an ester derivative thereof, a salt of either, or a solvate of any of these is from 1:1 to 1:10000.
(11) A medicine for lowering LDL cholesterol (LDL-C), obtained by combining a pharmaceutical composition comprising compound A, a salt thereof, or a solvate of either, and a pharmaceutically acceptable carrier; and a pharmaceutical composition comprising niacin, an ester derivative thereof, a salt of either, or a solvate of any of these, and a pharmaceutically acceptable carrier.
(12) The medicine described in (11), in which niacin is nicotinic acid.
(13) The medicine described in (11), in which niacin is nicotinic acid amide.
(14) The medicine described in any one of (11) to (13), in which a disease requiring lowering of LDL cholesterol (LDL-C) is hyper LDL cholesterolemia (LDL-C).
(15) The medicine described in any one of (11) to (14), in which a mass ratio between compound A, a salt thereof, or a solvate of either; and niacin, an ester derivative thereof, a salt of either, or a solvate of any of these is from 1:1 to 1:10000.
(16) A method for preventing and/or treating dyslipidemia of a patient, comprising: administering an effective amount of a pharmaceutical composition comprising compound A, a salt thereof, or a solvate of either, and niacin, an ester derivative thereof, a salt of either, or a solvate of any of these to a patient of dyslipidemia or a patient having a risk of suffering from dyslipidemia.
(17) The method described in (16), in which the dyslipidemia is hyper LDL cholesterolemia (LDL-C).
(18) The method described in (16) or (17), in which a mass ratio between compound A, a salt thereof, or a solvate of either, and niacin, an ester derivative thereof, a salt of either, or a solvate of any of these in the pharmaceutical composition is from 1:1 to 1:10000.
(19) The method described in any one of (16) to (18), in which niacin is nicotinic acid.
(20) A method for lowering LDL cholesterol (LDL-C) of a patient, comprising: administering an effective amount of a pharmaceutical composition comprising compound A, a salt thereof, or a solvate of either, and niacin, an ester derivative thereof, a salt of either, or a solvate of any of these to a patient requiring lowering of LDL cholesterol (LDL-C).
(21) The method described in (20), in which a disease requiring lowering of LDL cholesterol (LDL-C) is hyper LDL cholesterolemia (LDL-C).
(22) The method described in (20) or (21), in which niacin is nicotinic acid.
(23) A pharmaceutical composition for preventing and/or treating dyslipidemia, comprising, as an active ingredient, compound A, a salt thereof, or a solvate of either, which is used in combination with niacin, an ester derivative thereof, a salt of either, or a solvate of any of these.
(24) The pharmaceutical composition described in (23), in which the dyslipidemia is hyper LDL cholesterolemia (LDL-C).
(25) The pharmaceutical composition described in (23) or (24), in which a mass ratio between compound A, a salt thereof, or a solvate of either, and niacin, an ester derivative thereof, a salt of either, or a solvate of any of these is from 1:1 to 1:10000.
(26) The pharmaceutical composition described in any one of (23) to (25), in which niacin is nicotinic acid.
(27) A pharmaceutical composition for lowering LDL cholesterol (LDL-C), comprising, as an active ingredient, compound A, a salt thereof, or a solvate of either thereof, which is used in combination with niacin, an ester derivative thereof, a salt of either, or a solvate of any of these.
(28) The pharmaceutical composition described in (27), in which a disease requiring lowering of LDL cholesterol (LDL-C) is hyper LDL cholesterolemia (LDL-C).
(29) The pharmaceutical composition described in (27) or (28), in which niacin is nicotinic acid.
(30) Use of compound A, a salt thereof, or a solvate of either for manufacturing a pharmaceutical composition for preventing and/or treating dyslipidemia by combination use thereof with niacin, an ester derivative thereof, a salt of either, or a solvate of any of these.
(31) The use described in (29), in which the dyslipidemia is hyper LDL cholesterolemia (LDL-C).
(32) The use described in (30) or (31), in which a mass ratio between compound A, a salt thereof, or a solvate of either, and niacin, an ester derivative thereof, a salt of either, or a solvate of any of these is from 1:1 to 1:10000.
(33) The use described in any one of (30) to (32), in which niacin is nicotinic acid.
(34) Use of compound A, a salt thereof, or a solvate of either for manufacturing a pharmaceutical composition for lowering LDL cholesterol (LDL-C) by combination use thereof with niacin, an ester derivative thereof, a salt of either, or a solvate of any of these.
(35) The use described in (34), in which a disease requiring lowering of LDL cholesterol (LDL-C) is hyper LDL cholesterolemia (LDL-C).
(36) The use described in (34) or (35), in which niacin is nicotinic acid.
(37) A pharmaceutical composition for preventing and/or treating dyslipidemia, comprising, as an active ingredient, niacin, an ester derivative thereof, a salt of either or a solvate of any of these, which is used in combination with a pharmaceutical composition comprising compound A, a salt thereof, or a solvate of either, and a pharmaceutically acceptable carrier.
(38) The pharmaceutical composition described in (36), in which the dyslipidemia is hyper LDL cholesterolemia (LDL-C).
(39) The pharmaceutical composition described in (37) or (38), in which a mass ratio between niacin, an ester derivative thereof, a salt of either, or a solvate of any of these, and compound A, a salt thereof, or a solvate of either is from 1:1 to 10000:1.
(40) The pharmaceutical composition described in any one of (37) to (39), in which niacin is nicotinic acid.
(41) A pharmaceutical composition for lowering LDL cholesterol (LDL-C), comprising, as an active ingredient, niacin, an ester derivative thereof, a salt of either, or a solvate of any of these, which is used in combination with a pharmaceutical composition comprising compound A, a salt thereof, or a solvate of either, and a pharmaceutically acceptable carrier.
(42) The pharmaceutical composition described in (41), in which a disease requiring lowering of LDL cholesterol (LDL-C) is hyper LDL cholesterolemia (LDL-C).
(43) The pharmaceutical composition described in (41) or (42), in which niacin is nicotinic acid.
(44) Use of niacin, an ester derivative thereof, a salt of either, or a solvate of any of these for manufacturing a pharmaceutical composition for preventing and/or treating dyslipidemia by combination use thereof with compound A, a salt thereof, or a solvate of either.
(45) The use described in (43), in which the dyslipidemia is hyper LDL cholesterolemia (LDL-C).
(46) The use described in (44) or (45), in which a mass ratio between niacin, an ester derivative thereof, a salt of either, or a solvate of any of these, and compound A, a salt thereof, or a solvate of either is from 1:1 to 10000:1.
(47) The use described in any one of (44) to (46), in which niacin is nicotinic acid.
(48) Use of niacin, an ester derivative thereof, a salt of either, or a solvate of any of these for manufacturing a pharmaceutical composition for lowering LDL cholesterol (LDL-C) by combination use with compound A, a salt thereof, or a solvate of either.
(49) The use described in (48), in which a disease requiring lowering of LDL cholesterol (LDL-C) is hyper LDL cholesterolemia (LDL-C).

### Effects of the Invention

The pharmaceutical composition and the medicine according to the present invention exhibit an excellent effect of lowering LDL cholesterol in blood, and are useful for preventing and/or treating dyslipidemia, particularly hyper LDL cholesterolemia.

### Brief Description of Drawings

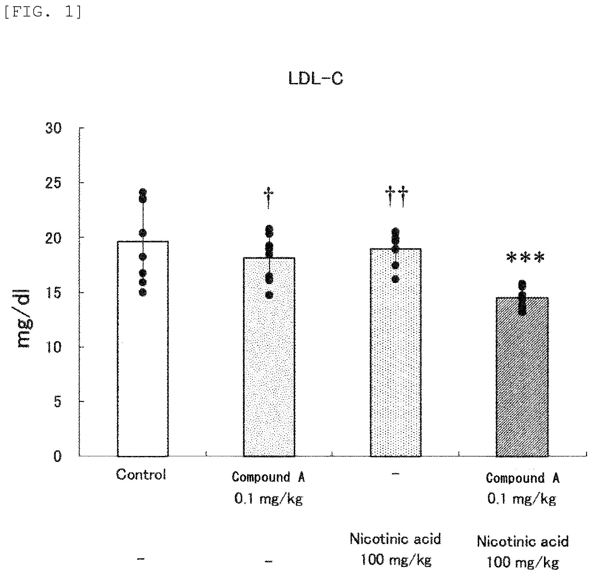

FIG. 1 illustrates LDL-C in plasma at the time of use of compound A (0.1 mg/kg) alone, use of nicotinic acid (100 mg/kg) alone, and combination use of compound A (0.1 mg/kg) and nicotinic acid (100 mg/kg).

For example, compound A used in the present invention can be produced in accordance with a method described in WO 2005/023777 A1. In addition, compound A can be produced in accordance with a method described in literature.

A chemical structural formula of compound A is as follows.

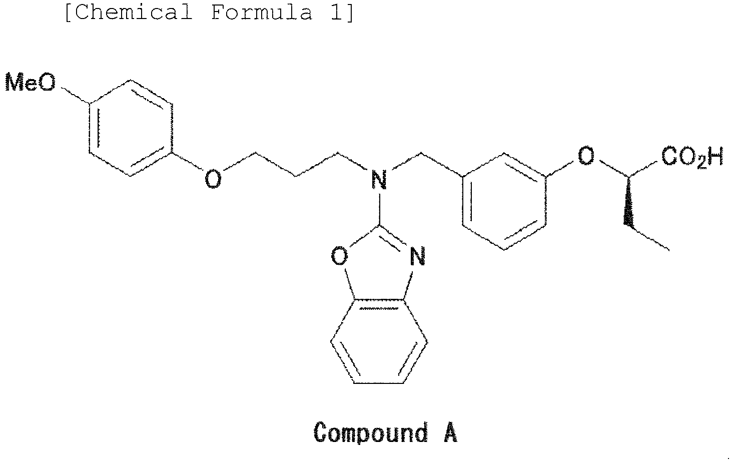

In addition, the present invention can use a salt of compound A or a solvate thereof. The salt and the solvate can be manufactured by a usual method.

The salt of compound A is not particularly limited as long as being pharmaceutically acceptable. However, examples thereof include an alkali metal salt such as a sodium salt or a potassium salt; an alkaline earth metal salt such as a calcium salt or a magnesium salt; an organic base salt such as an ammonium salt or a trialkylamine salt; a mineral acid salt such as a hydrochloride or a sulfate; and an organic acid salt such as an acetate.

Examples of the solvate of compound A or a salt thereof include a hydrate and an alcohol solvate (for example, ethanol solvate).

Niacin is a compound also referred to as pyridine-3-carboxylic acid (nicotinic acid), pyridine-3-carboxylic acid amide (nicotinic acid amide), or vitamin B₃. Examples of niacin in the present invention include nicotinic acid and nicotinic acid amide. Examples of an ester derivative of niacin include niceritrol. These compounds are also included in a concept of the present invention.

Chemical structural formulae of nicotinic acid, nicotinic acid amide, and niceritrol are as follows.

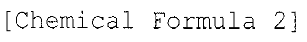

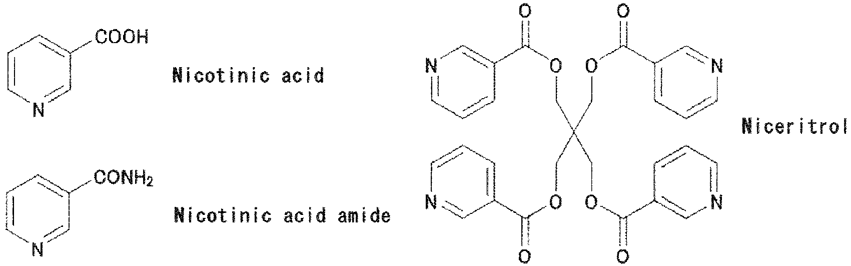

The salt of niacin or an ester derivative of niacin is not particularly limited as long as being pharmaceutically acceptable. However, examples thereof include an alkali metal salt such as a sodium salt or a potassium salt; an alkaline earth metal salt such as a calcium salt or a magnesium salt; an organic base salt such as an ammonium salt or a trialkylamine salt; a mineral acid salt such as a hydrochloride or a sulfate; and an organic acid salt such as an acetate.

Examples of the solvate of compound A or a salt thereof include a hydrate and an alcohol solvate (for example, ethanol solvate).

As described in Examples below, by combination use of compound A, a salt thereof, or a solvate of either, and nicotinic acid, nicotinic acid amide, which are collectively referred to as niacin, an ester derivative thereof, or a salt thereof, a particularly strong effect of lowering LDL-C was exhibited in an evaluation system using a SD rat. Therefore, the pharmaceutical agents of the present invention are useful for preventing and/or treating dyslipidemia such as hypercholesterolemia or hyper LDL cholesterolemia.

When the term dyslipidemia is used here, dyslipidemia means a case where any one of a total triglyceride (TG) level, a total cholesterol (TC) level, a VLDL cholesterol (VLDL-C) level, a LDL cholesterol (LDL-C) level, and a HDL cholesterol (HDL-C) level in blood, or two or more thereof deviate from a range of normal values. A case where the LDL cholesterol (LDL-C) level deviates from a range of normal values is a preferable example of dyslipidemia in the present invention. A disease requiring lowering of LDL cholesterol (LDL-C) in the present invention means a case where the LDL-C level in blood is higher than a normal value.

The pharmaceutical composition of the present invention can be formed into a dosage form such as a tablet, a capsule, a granule, a powder, a lotion, an ointment, an injection, or a suppository singly or using another pharmaceutically acceptable carrier. These formulations can be manufactured by a known method. For example, when a formulation for oral administration is manufactured, the formulation can be manufactured by appropriately combining and formulating a dissolving agent such as gum tragacanth, gum arabic, a sucrose fatty acid ester, lecithin, olive oil, soybean oil, or PEG400; an excipient such as starch, mannitol, or lactose; a binder such as methyl cellulose, sodium carboxymethylcellulose, or hydroxypropylcellulose; a disintegrating agent such as crystalline cellulose or calcium carboxymethylcellulose; a lubricant such as talc or magnesium stearate; and a flow improver such as light anhydrous silicic acid.

As a use form of the pharmaceutical composition of the present invention, it is possible to use a form in which an effect for preventing and/or treating dyslipidemia such as hypercholesterolemia or hyper LDL cholesterolemia is obtained by combining a) compound A, a salt thereof, or a solvate of either, and b) nicotinic acid and nicotinic acid amide collectively referred to as niacin, an ester derivative thereof, a salt of either, or a solvate of any of these, and by using a synergistic effect for raising HDL-C in blood due to administration of the two pharmaceutical agents in addition to an effect by each of the pharmaceutical agents. However, the use form of the present invention is not limited thereto. Compound A, a salt thereof, or a solvate of either, and nicotinic acid , nicotinic acid amide, which are collectively referred to as niacin, an ester derivative thereof, a salt of either, or a solvate of any of these may be administered simultaneously, or may be administered separately at an interval.

Compound A, a salt thereof, or a solvate of either, and nicotinic acid, nicotinic acid amide, which are collectively referred to as niacin, an ester derivative thereof, a salt of either, or a solvate of any of these may be formulated into a single formulation, or the two pharmaceutical agents may be formulated separately to be used as a kit. That is, the pharmaceutical composition of the present invention may be a kit formed by combining a pharmaceutical agent comprising at least one selected from compound A, a salt thereof, and a solvate of either as an active ingredient, and/or a pharmaceutical agent comprising at least one of the solvates.

In the present invention, when the two pharmaceutical agents are administered as a single formulation, a blending ratio between compound A, a salt thereof, or a solvate of either, and nicotinic acid, nicotinic acid amide, which are collectively referred to as niacin, an ester derivative thereof, a salt of either, or a solvate of any of these can be appropriately selected in a range of an effective dose of each active ingredient, but in general, is preferably from 1:1 to 1:10000, more preferably from 1:5 to 1:4000, and particularly preferably from 1:10 to 1:2000 in terms of a mass ratio as nicotinic acid.

When compound A, a salt thereof, or a solvate of either, and nicotinic acid, nicotinic acid amide, which are collectively referred to as niacin, an ester derivative thereof, a salt of either, or a solvate of any of these are formulated separately, dosage forms of the two pharmaceutical agents may be the same as or different from each other. In addition, the numbers of dose for ingredients may be different from one another.

Compound A, a salt thereof, or a solvate of either of the present invention is administered orally or parenterally. The dose of the pharmaceutical agents of the present invention vary according to a patient's weight, age, sex, symptoms, and the like. However, in a case of an adult, it is usually desirable to administer 0.001 to 100 mg, preferably 0.01 to 10 mg, and particularly preferably 0.1 to 0.4 mg as compound A per day in one to three parts. As for nicotinic acid or a solvate thereof, it is desirable to administer 0.01 to 800 mg, preferably 0.1 to 400 mg, and particularly preferably 1 to 100 mg as nicotinic acid per day in one to three parts.

### [Examples]

Hereinafter, the present invention will be described more specifically based on Examples, but the present invention is not limited in any way by the Examples.

### Example 1: Effect on LDL-C of rat in combination use of compound A and niacin

### 1. Method

Male rats (6-week-old, Jcl: SD, CLEA Japan, Inc.) were used for an experiment. Blood was collected from the jugular vein under satiation. The rats were divided into four groups (N = 8) based on TG and TC in plasma and a body weight. For one week from the next day, each of a solvent (0.5% methylcellulose aqueous solution: MC), compound A alone, nicotinic acid alone, and combination of compound A and nicotinic acid was orally administered once per day. Four hours after final administration of the pharmaceutical agent, blood was collected under isoflurane anesthesia, and LDL-C in plasma was measured using high performance liquid chromatography by a method of Usui et al (Usui S et al. Clin Chem., 46, 63-72, 2000).

### 2. Group configuration

Group 1: Control
Group 2: 0.1 mg/kg of compound A
Group 3: 100 mg/kg of nicotinic acid
Group 4: 0.1 mg/kg of compound A and 100 mg/kg of nicotinic acid

### 3. Statistical analysis and data processing method

Results were presented by an average value ± standard deviation. Comparison between the control group and each of the pharmaceutical agent administration groups was performed by a multiple comparison test of Dunnett, and a risk rate of less than 5% was determined to have a significant difference.

### 4. Result

FIG. 1 illustrates results of measurement of LDL-C. Compound A alone or nicotinic acid alone did not exhibit a clear effect on LDL-C, but combination use thereof exhibited a 26% lowering effect. The combination use group exhibited a significant lowering effect compared not only with the Control group but also with the single pharmaceutical agent administration groups (Tukey's test). Therefore, it has been revealed that combined administration of the two compounds exhibits a strong effect of lowering lipid specifically on LDL-C.

The above results indicate that combination use of compound A and nicotinic acid becomes effective even with a dose with which a therapeutic effect of the pharmaceutical agent is not observed by single administration. That is, it has been indicated that combination use of compound A and nicotinic acid which are the pharmaceutical and the drug composition according to the present invention exhibits a strong action for improving dyslipidemia.

### Industrial Applicability

The pharmaceutical composition and the medicine according to the present invention exhibit an excellent effect of lowering LDL-C in blood, are useful for preventing and/or treating dyslipidemia, particularly hyper LDL cholesterolemia, and therefore have industrial applicability.

## Claims

1. A pharmaceutical composition for preventing and/or treating dyslipidemia, comprising:
(R)-2-[3-[[N-(benzoxazol-2-yl)-N-3-(4-methoxyphenoxy)pr opyl]aminomethyl]phenoxy] butyric acid, a salt thereof, or a solvate of either; and
niacin, an ester derivative thereof, a salt of either, or a solvate of any of these.

2. The pharmaceutical composition according to claim 1, wherein niacin is nicotinic acid.

3. The pharmaceutical composition according to claim 1, wherein niacin is nicotinic acid amide.

4. The pharmaceutical composition according to any one of claims 1 to 3, wherein the dyslipidemia is hyper LDL cholesterolemia.

5. The pharmaceutical composition according to any one of claims 1 to 4, wherein a mass ratio between (R)-2-[3-[[N-(benzoxazol-2-yl)-N-3-(4-methoxyphenoxy)propyl ]aminomethyl]phenoxy] butyric acid, a salt thereof, or a solvate of either; and nicotinic acid, nicotinic acid amide, which are collectively referred to as niacin, an ester derivative thereof, a salt of either, or a solvate of any of these is from 1:1 to 1:10000.

6. A medicine for lowering LDL cholesterol, obtained by combining:
a pharmaceutical composition comprising (R)-2-[3-[[N-(benzoxazol-2-yl)-N-3-(4-methoxyphenoxy)propyl ]aminomethyl]phenoxy] butyric acid, a salt thereof, or a solvate of either, and a pharmaceutically acceptable carrier; and
a pharmaceutical composition comprising nicotinic acid, nicotinic acid amide, which are collectively referred to as niacin, an ester derivative thereof, a salt of either, or a solvate of any of these, and a pharmaceutically acceptable carrier.

7. The medicine according to claim 6, wherein a disease requiring lowering of LDL-C is hyper LDL cholesterolemia.

8. The medicine according to claim 6 or 7, wherein a mass ratio between (R)-2-[3-[[N-(benzoxazol-2-yl)-N-3-(4-methoxyphenoxy)propyl ]aminomethyl]phenoxy] butyric acid, a salt thereof, or a solvate of either; and nicotinic acid, nicotinic acid amide, which are collectively referred to as niacin, an ester derivative thereof, a salt of either, or a solvate of any of these is from 1:1 to 1:10000.
